# FASCICULE DE BREVET EUROPEEN

(11) **EP 2 854 911 B1**
(45) Date de publication et mention de la délivrance du brevet: **04.05.2016**
(21) Numéro de dépôt: 13731838.2
(22) Date de dépôt: 30.05.2013
(51) Int. Cl.: A61M 15/00

(54) **INHALATEUR DE POUDRE SÈCHE**
TROCKENPULVERINHALATOR
DRY POWDER INHALATOR

(30) Priorité: 31.05.2012 FR 1255009
(43) Date de publication de la demande: 08.04.2015
(73) Titulaire: Aptar France SAS, 27110 Le Neubourg (FR)
(72) Inventeur: COLOMB, Arnaud, 78480 Verneuil Sur Seine (FR); BAILLET, Matthieu, 76240 Bonsecours (FR); KIRNIAK, Maxime, 76000 Rouen (FR); LAUT, Antoine, 95420 Wy Dit Joli Village (FR)
(74) Mandataire: CAPRI
(86) Numéro de dépôt international: PCT/FR2013/051213
(87) Numéro de publication internationale: WO 2013/178949

(56) Documents cités:
- WO-A1-2006/079750
- WO-A2-2008/012458
- FR-A1- 2 909 645
- FR-A1- 2 918 353

## Description

La présente invention concerne un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. Un autre type d'inhalateur consiste à disposer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est d'abord chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a chargé la dose (soit à partir d'un réservoir multidoses, soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Un autre problème qui peut se poser concerne l'assemblage de certaines pièces, notamment mobiles, qui doivent supporter des contraintes importantes en fonctionnement, et pour lesquels l'assemblage doit être particulièrement fiable pour éviter tout risque de dysfonctionnement. Avec la petite taille de certaines pièces, il peut être compliqué de garantir une telle fiabilité d'assemblage. Un autre problème peut se poser avec les dispositifs utilisant des moyens de perçage pour ouvrir un réservoir individuel, tel qu'un blister, à chaque actionnement. Pour éviter des variations de performances, il est souhaitable que le perçage soit toujours réalisé sensiblement au même endroit du blister, si possible en son centre. Il est donc souhaitable de pouvoir prédéfinir avec précision la position des blisters par rapport aux moyens de perçage, afin de garantir une constance des performances. Le document WO 2008/012458 décrit un dispositif de l'art antérieur.

La présente invention a pour but de fournir un inhalateur de poudre sèche qui ne reproduit pas les inconvénients susmentionnés.

En particulier, la présente invention a pour but de fournir un tel inhalateur qui soit simple et peu coûteux à fabriquer et à assembler, fiable à l'assemblage et en utilisation, garantissant une précision et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

La présente invention a donc pour objet un inhalateur de poudre sèche, comportant un corps principal, ledit inhalateur comportant une pluralité de réservoirs individuels, tels que des blisters, contenant chacun une dose unique de poudre, et disposés les uns derrière les autres sur une bande souple, des moyens d'ouverture étant prévus pour ouvrir un réservoir individuel à chaque actionnement de l'inhalateur, ledit inhalateur comportant des premiers moyens de déplacement adaptés à amener un réservoir individuel en face desdits moyens d'ouverture avant et/ou après chaque actionnement, et des seconds moyens de déplacement adaptés à déplacer un réservoir individuel contre lesdits moyens d'ouverture à chaque actionnement, lesdits premiers moyens de déplacement comportant une roue d'indexage pourvue d'au moins un évidement recevant un réservoir individuel, ledit évidement étant défini par une paroi de fond et une paroi latérale interne de ladite roue d'indexage, et par une paroi latérale externe formée sur un élément de réglage rapporté assemblé sur ladite roue d'indexage.

Avantageusement, ladite roue d'indexage comporte une pluralité d'évidements.

Avantageusement, ledit élément de réglage rapporté comporte un manchon central et une paroi externe annulaire, reliée audit manchon central par une pluralité d'entretoises, lesdites entretoises formant lesdites parois latérales externes desdits évidements de la roue d'indexage.

Avantageusement, les réservoirs individuels sont des blisters, et après assemblage de l'élément de réglage rapporté sur la roue d'indexage, la dimension des évidements est sensiblement égale ou légèrement supérieure à la dimension extérieure des cavités des blisters.

Avantageusement, l'inhalateur comporte un système de déclenchement par l'inhalation qui comporte une chambre d'air déformable coopérant avec un embout d'inhalation, et un élément déclencheur coopérant avec ladite chambre d'air, de sorte que lors d'une inhalation à travers ledit embout d'inhalation, ladite chambre d'air est déformée et ledit élément déclencheur actionne lesdits moyens d'ouverture, de sorte que lors d'une inhalation à travers l'embout d'inhalation, un réservoir est ouvert par lesdits moyens d'ouverture.

Avantageusement, lesdits seconds moyens de déplacement comportent des moyens de support mobiles déplaçables entre une position de non-distribution et une position de distribution, lesdits moyens de support mobiles étant sollicités vers leur position de distribution par des moyens élastiques, tel qu'un ressort ou une lame élastique, et étant retenus en position de non-distribution par des moyens de blocage qui sont libérés par l'inhalation de l'utilisateur.

Avantageusement, ladite roue d'indexage est montée rotative sur un axe desdits moyens de support mobiles.

Avantageusement, lesdits moyens d'ouverture comportent un élément de perçage fixe par rapport audit corps principal, adapté à découper une paroi de fermeture du réservoir de telle sorte que la ou les partie(s) découpée(s) n'obstrue(nt) pas la ou les ouverture(s) formée(s).

Avantageusement, l'inhalateur comporte au moins un élément de capot monté pivotant sur ledit corps principal entre une position fermée et une position ouverte.

Ces caractéristiques et avantages et d'autres de la présente invention apparaîtront plus clairement au cours de la description détaillée suivante, faite en référence aux dessins joints, donnés à titre d'exemples non limitatifs, sur lesquels
- les figures 1 à 3 sont des vues schématiques en section transversale d'un inhalateur de poudre sèche selon un mode de réalisation avantageux de l'invention, respectivement avant ouverture, après ouverture mais avant inhalation, et après inhalation,
- la figure 4 est une vue schématique de détail en section transversale d'une partie de l'inhalateur des figures 1 à 3, et
- la figure 5 est une vue schématique en perspective de l'élément de réglage rapporté.

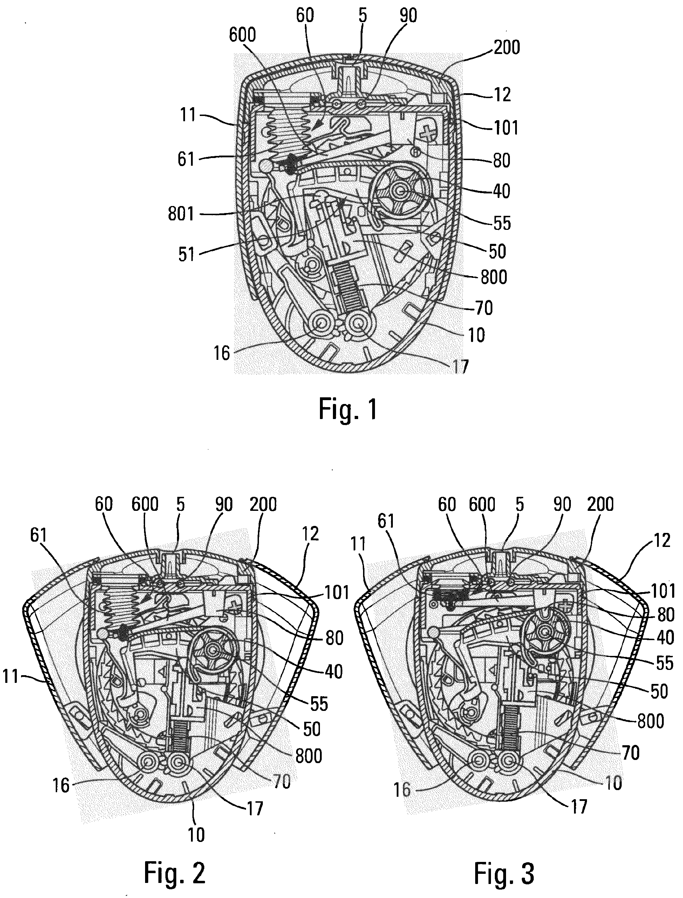

Sur les figures 1 à 3, il est représenté un exemple avantageux d'un inhalateur de poudre sèche. Cet inhalateur comporte un corps principal 10 sur lequel peuvent être montées coulissantes deux parties 11, 12 formant capot, adaptées à être ouvertes pour ouvrir et charger le dispositif. Le corps principal 10 peut être de forme environ arrondie comme représenté sur les figures, mais il pourrait avoir toute autre forme appropriée. Un corps supérieur 101 est assemblé au corps principal 10, et un embout buccal 200 est assemblé sur ledit corps supérieur 101. Cet embout buccal 200 définit un orifice de distribution 5 à travers lequel l'utilisateur va inhaler lors de l'actionnement du dispositif. Cet orifice de distribution 5 est typiquement disposé environ au centre de l'embout buccal 200. Les capots 11, 12 peuvent s'ouvrir par pivotement autour d'un axe de rotation commun, ou autour de deux axes parallèles en étant engrené l'un avec l'autre. Tout autre moyen d'ouverture du dispositif est envisageable. En variante, le dispositif pourrait ne comporter qu'un seul capot au lieu de deux.

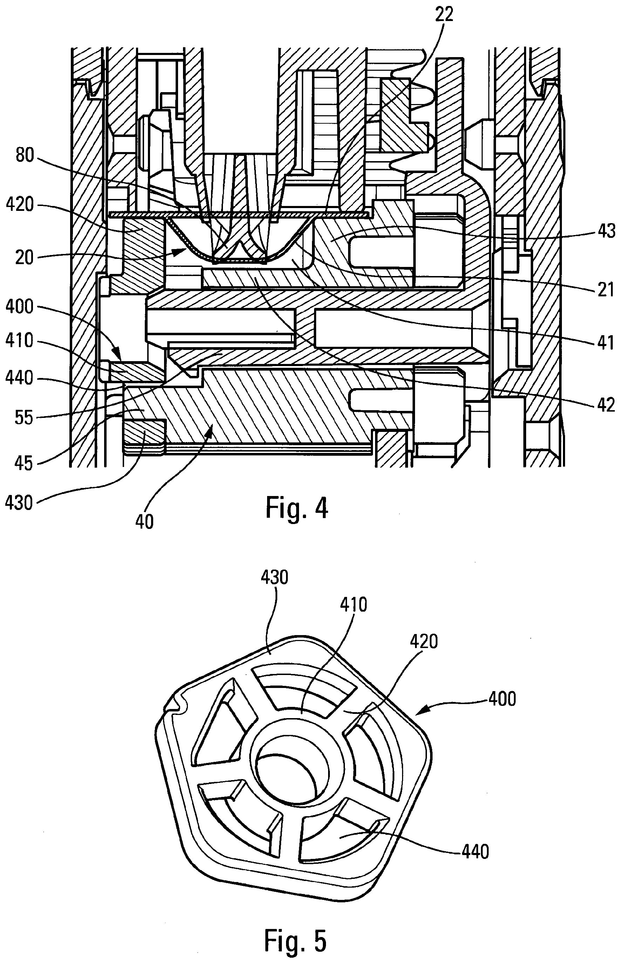

A l'intérieur du corps principal 10, il est prévu une bande de réservoirs individuels (non représentée dans des buts de clarté), également appelés blisters, réalisée sous forme d'une bande allongée sur laquelle les blisters sont disposés les uns derrière les autres, de manière connue. Un blister 20 est représenté sur la figure 4. Cette bande de blisters est avantageusement constituée d'une couche ou paroi de base formant les cavités 21 recevant les doses de poudre, et d'une couche ou paroi de fermeture 22 qui obture de manière étanche chacun desdits blisters. Avant la première utilisation, la bande de blisters peut être enroulée à l'intérieur du corps principal 10, de préférence dans une partie de stockage, et des premiers moyens de déplacement de bande 40, notamment rotatifs, sont prévus pour progressivement dérouler et faire avancer cette bande de blisters.

Des seconds moyens de déplacement 50, notamment pivotants sur le corps principal 10, sont prévus pour amener un blister respectif dans une position de distribution à chaque actionnement du dispositif. Ces seconds moyens de déplacement sont avantageusement pivotants entre une position de non-distribution et une position de distribution, dans laquelle un blister coopère avec lesdits moyens d'ouverture.

La partie de bande comportant les blisters vides est avantageusement adaptée à s'enrouler dans un autre endroit dudit corps 10, de préférence une partie de réception, comme cela sera décrit plus en détail ci-après.

L'inhalateur comporte des moyens d'ouverture de blister 80 comportant de préférence une aiguille de perçage et/ou de coupage de la couche de fermeture des blisters. De préférence, les moyens d'ouverture comportent un élément de perçage 80, fixe par rapport aux corps principal 10 et supérieur 101, et contre lequel un blister respectif 20 est déplacé à chaque actionnement par les seconds moyens de déplacement. Le blister est alors percé par ledit élément de perçage, qui pénètre dans ledit blister pour expulser la poudre au moyen du flux d'inhalation de l'utilisateur. Cette position est illustrée sur la figure 4. Avantageusement, l'élément de perçage est adapté à découper une paroi de fermeture du réservoir de telle sorte que la ou les partie(s) découpée(s) n'obstrue(nt) pas la ou les ouverture(s) formée(s). Les documents WO 2006/079750 et WO 2009/007640 décrivent de tels moyens d'ouverture de blister.

Les premiers moyens de déplacement 40 sont adaptés à faire avancer la bande de blisters après chaque inhalation de l'utilisateur. Les seconds moyens de déplacement 50 sont adaptés à déplacer le blister à vider contre lesdits moyens d'ouverture 80 lors de l'actionnement, avant chaque inhalation. Ces seconds moyens de déplacement 50 peuvent être sollicités par un élément élastique 70, tel qu'un ressort ou tout autre élément élastique équivalent, ledit élément élastique pouvant être pré-chargé lors de l'ouverture du dispositif.

Les premiers moyens de déplacement 40 sont formés par une roue d'indexage qui reçoit et guide la bande de blisters. La suite de la description sera donc faite en référence à une telle roue d'indexage 40. Une rotation de cette roue d'indexage 40 fait avancer la bande de blisters. Avant chaque inhalation, un blister plein est toujours en position face aux moyens d'ouverture 80. Les seconds moyens de déplacement 50 comportent un organe pivotant, ladite roue d'indexage 40 étant avantageusement montée rotative sur un axe 55 dudit organe pivotant 50.

Un cycle d'actionnement du dispositif peut être le suivant. Lors de l'ouverture du dispositif, les deux parties latérales 11, 12 formant capot sont écartées l'une de l'autre en pivotant sur le corps pour ouvrir le dispositif, et ainsi charger le dispositif. Dans cette position la roue d'indexage 40 ne peut pas se déplacer vers l'élément de perçage 80 car les seconds moyens de déplacement 50 sont retenus par des moyens de blocage appropriés (non représentés dans des buts de clarté). Les documents WO 2009/077700 et WO 2009/136098 décrivent de tels moyens de blocage. Lors de l'inhalation par l'utilisateur à travers l'embout buccal, ces moyens de blocage sont débloqués, ce qui provoque alors le déplacement de ladite roue d'indexage 40 en direction de l'aiguille, et donc l'ouverture d'un blister.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture, on prévoit un système de déclenchement par l'inhalation 60 qui comporte avantageusement une chambre d'air 61 déformable sous l'effet de l'inhalation, cette chambre d'air étant adaptée à libérer les moyens de blocage. La chambre d'air 61 peut avantageusement être réalisée en forme de soufflet. L'inhalation de l'utilisateur provoque la déformation de ladite chambre d'air déformable, libérant ainsi lesdits moyens de blocage et permettant donc le déplacement des seconds moyens de déplacement, et donc d'un blister respectif, vers sa position d'ouverture. Le blister n'est donc ouvert qu'au moment de l'inhalation, de sorte qu'il est simultanément vidé. Il n'y a donc aucun risque de perte de dose entre l'ouverture du blister et son vidage.

L'inhalateur comporte en outre une chambre de dispersion 90 qui est destinée à recevoir la dose de poudre après ouverture d'un blister respectif. Avantageusement, cette chambre de dispersion est pourvue d'au moins une bille, de préférence plusieurs, qui se déplace(nt) à l'intérieur de ladite chambre 90 pendant l'inhalation, notamment pour améliorer la distribution du mélange air et poudre après ouverture d'un blister, afin d'augmenter l'efficacité du dispositif.

Il peut être avantageux que les moyens d'ouverture, en particulier l'élément de perçage, soient directement reliés à ladite chambre de dispersion, par exemple via un canal menant à ladite chambre 90.

Après l'inhalation, lorsque l'utilisateur referme le dispositif, tous les composants reviennent vers leurs positions de repos initiales. Le dispositif est alors prêt pour un nouveau cycle d'utilisation.

Selon un aspect avantageux de l'inhalateur, les blisters sont formés sur une bande allongée souple, qui, au début, est principalement stockée sous forme de bobine dans un logement de stockage à l'intérieur du corps principal 10 du dispositif. Avantageusement, cette bande de blisters enroulée est maintenue par des parois internes dudit logement de stockage sans que son extrémité arrière (dans le sens d'avancement de la bande de blisters) ne soit fixée par rapport audit corps principal 10, ce qui permet un assemblage plus aisé de cette bobine de bande de blisters à l'intérieur du dispositif. La bande de blisters est déplacée au moyen de la roue d'indexage 40 qui présente avantageusement au moins un, de préférence plusieurs évidements. Ainsi, lorsque cette roue d'indexage 40 tourne, elle fait avancer la bande de blisters. Bien entendu, de manière additionnelle, on pourrait utiliser d'autres moyens d'avancée de bande de blisters, par exemple en prévoyant un profil sur les bords longitudinaux latéraux de la bande de blisters, ledit profil étant adapté à coopérer avec des moyens d'entraînement appropriés. De même, des trous ménagés le long des bords latéraux de la bande de blisters pourraient également être utilisés pour faire avancer la bande de blisters au moyen de roues dentées coopérant avec lesdits trous.

Après ouverture d'un ou plusieurs blister(s), la partie de bande de blisters avec les blisters vidés doit pouvoir être stockée de manière aisée et non encombrante dans le dispositif, en évitant tout risque de blocage. Avantageusement, cette bande de blisters usée s'enroule automatiquement sur elle-même pour à nouveau former une bobine.

Selon encore un autre aspect de l'inhalateur, un dispositif de comptage ou d'indication de doses (non représenté dans des buts de clarté) peut également être prévu. Ce dispositif peut comporter des chiffres ou symboles inscrits directement sur la bande de blister et visibles à travers une fenêtre appropriée dans le corps principal 10 du dispositif. En variante, on pourrait envisager d'utiliser un compteur avec un ou plusieurs disque(s) ou anneau(x) rotatif(s) comportant des numéros ou symboles. Les documents WO 2008/012458 et WO 2011/154659 décrivent de tels compteurs. Afin d'éviter de compter des doses qui ne sont pas distribuées, par exemple en cas d'erreur de manipulation, ou d'une manipulation incomplète du dispositif, il est souhaitable que le compteur ou indicateur ne soir actionné qu'une fois que l'utilisateur a inhalé, puisque c'est cette inhalation qui conditionne l'ouverture et la distribution de la dose de chaque blister. Avantageusement, l'actionnement du compteur se fait donc après inhalation, lorsque l'utilisateur referme le dispositif.

L'élément de capot mobile 12 est solidaire d'un organe d'armement 800 qui peut coulisser dans un logement approprié. Cet organe d'armement 800 est avantageusement pivotant par rapport audit corps 10 ensemble avec l'élément de capot 12. Cet organe d'armement 800 peut se déplacer contre le ressort 70, avantageusement un ressort à boudins, disposé dans ledit logement. L'organe d'armement 800 est donc connecté d'un côté audit ressort 70 et de l'autre côté, il coopère avec les seconds moyens de déplacement, en particulier avec un organe 50 pivotant sur le corps 10, et sur lequel est fixée de manière rotative la roue d'indexage 40.

Lorsque l'élément de capot mobile 12 est ouvert, l'organe d'armement 800 est déplacé dans son logement en comprimant le ressort 70. L'organe pivotant 50 des seconds moyens de déplacement est lui empêché de se déplacer par les moyens de blocage susmentionnés, qui ne seront libérés qu'au moment de l'inhalation. Ainsi, en l'absence d'inhalation dans la position ouverte, la fermeture des éléments de capot 11, 12 provoquerait simplement le retour à la position de repos pour l'organe d'armement 800, et la décompression du ressort 70.

Ainsi, lorsque l'utilisateur ouvre l'inhalateur il charge le système. S'il n'inhale pas et qu'il referme l'inhalateur, celui-ci reprend simplement sa position de départ sans déplacer la bande de blisters ni les moyens de blocage. Il n'y a donc aucun risque qu'un blister (et donc une dose de produit actif) soit perdu par un actionnement malencontreux ou incomplet dans lequel l'utilisateur n'exercerait pas d'inhalation entre l'ouverture et la fermeture. L'ouverture du blister, son vidage, la distribution de la poudre dans les poumons de l'utilisateur, le déplacement de la bande de blisters pour amener un nouveau blister plein en face des moyens d'ouverture ainsi que le comptage de la dose ne sont donc possibles que si l'utilisateur inhale.

Les moyens de blocage, qui bloquent les seconds moyens de déplacement et notamment l'organe pivotant qui coopère avec l'organe d'armement, sont connectés à la chambre d'air déformable 61 sensible à l'inhalation de l'utilisateur via un élément déclencheur 600, de sorte que lors de l'inhalation de l'utilisateur, ladite chambre d'air déformable se déforme, faisant pivoter ledit élément déclencheur 600 et libérant lesdits moyens de blocage. Ceci permet le déplacement desdits seconds moyens de déplacement vers leur position de distribution sous l'effet de la force exercée par le ressort comprimé 70 sur l'organe d'armement 800 qui pousse sur l'organe pivotant 50. Ce déplacement provoque l'ouverture d'un blister plein et la distribution d'une dose.

Une surface de came 51 est formée sur lesdits moyens de support mobiles 50, sur laquelle glisse l'organe d'armement 800. L'organe d'armement 800 est donc adaptée à comprimer le ressort 70 lorsque l'élément de capot 12 est ouvert, et à décomprimer ledit ressort 70 lorsque ledit élément de capot 12 est refermé.

Avantageusement, l'organe d'armement 800 comporte, dans sa partie en contact avec la surface de came 51, une partie arrondie 801 pour favoriser le glissement de l'organe d'armement 800 sur ladite surface de came 51.

Les moyens de support mobiles sont dans ce mode de réalisation réalisés sous la forme d'un organe 50 monté pivotant sur le corps 10 autour d'un axe de pivotement. La surface de came précitée 51 étant formée sur ledit organe pivotant 50, lorsque le ressort 70 est chargé lors de l'ouverture de l'élément de capot mobile 12, ledit organe pivotant 50 est sollicité vers sa position de distribution par ledit organe d'armement 800 et le ressort 70 comprimé.

La surface de came 51 peut comporter au moins deux parties de pente différentes, avantageusement séparées par un sommet. En partant de la position fermée de l'élément de capot mobile, une partie de chargement sur laquelle coulisse l'organe d'armement 800 permet la compression du ressort 70 comme décrit précédemment. Lorsque le ressort est chargé, c'est-à-dire comprimé, la surface de came 51 peut prévoir une seconde partie de pente différente avec laquelle l'organe d'armement 800 va coopérer en position d'ouverture du dispositif. De préférence, l'organe d'armement 800 exerce une force sensiblement perpendiculaire sur cette seconde partie de surface de came. De cette manière, la position chargée est stable. En variante, la seconde partie de pente peut former un cran de butée dans lequel l'organe d'armement 800 vient se positionner en position ouverte.

Après l'inhalation, c'est-à-dire en position de distribution, les moyens de blocage ont été libérés et les moyens de support mobiles 50 ont été déplacés vers le haut par le ressort comprimé 70.

Avantageusement, les deux éléments de capot mobiles 11, 12 sont engrenés l'un dans l'autre via des engrenages appropriés pour garantir une ouverture et une fermeture symétrique desdits deux éléments de capot mobiles. Cet engrenage peut se faire à proximité de leur axe de pivotement 16,17.

La roue d'indexage 40 comporte au moins un, avantageusement cinq, évidements 41, destinés à recevoir une cavité 21 d'un blister 20 respectif.

Les évidements 41 sont avantageusement définis par une paroi de fond 42 et une paroi latérale interne 43, qui forment partie intégrante de la roue d'indexage 40.

Une paroi latérale externe 420 des évidements 41 est, selon l'invention, formée par un élément de réglage rapporté 400.

Ainsi, lors de l'assemblage, la bande de blisters est assemblée sur la roue d'indexage 40, en disposant au moins une cavité 21 d'un blister 20 dans un évidement 41, avec la paroi de fermeture 22 du blister 20 s'étendant à l'extérieur dudit évidement 41. L'élément de réglage rapporté 400 est alors assemblé sur la roue d'indexage 40, de telle sorte que la paroi latérale externe 420 de l'évidement définit une dimension d'évidement 41 sensiblement égale ou légèrement supérieure à la dimension extérieure de la cavité 21 du blister. De cette manière, toutes les cavités seront positionnées sensiblement dans la même position sur la roue d'indexage 40, avec ainsi un perçage reproductible réalisé sensiblement au même endroit du blister 20 à chaque actionnement. De plus, il n'y a pas de risques de décalage de la bande de blister par rapport à la roue d'indexage, puisque l'élément de réglage rapporté vient positionner précisément la cavité 21 du blister dans l'évidement 41.

Avantageusement, l'élément de réglage rapporté 400 comporte un manchon creux central 410 qui peut recevoir l'axe 55 de l'organe pivotant 50. Une paroi externe annulaire 430, cylindrique ou de forme extérieure polygonale comme visible sur la figure 5, est reliée audit manchon central creux 410 par des entretoises 420, qui après assemblage de l'élément de réglage rapporté 400 sur la roue d'indexage 40, forment les parois latérales externes des évidements 41. Une ou plusieurs des ouvertures 440, formées entre le manchon central 410, la paroi externe annulaire 430 et les entretoises 420, peuvent recevoir des projections 45 de la roue d'indexage, pour assurer l'assemblage de l'élément de réglage rapporté 400 sur la roue d'indexage 40.

La présente invention permet donc de fournir un inhalateur de poudre sèche qui procure notamment les fonctions suivantes :
▪ une pluralité de doses individuelles de poudre stockées dans des blisters individuels étanches, par exemple 30 ou 60 doses stockées sur une bande enroulée en bobine ;
▪ la poudre libérée au moyen d'un perçage actionné par l'inhalation de l'utilisateur, ce perçage du blister étant réalisé au moyen d'un système de détection d'inhalation couplé à un système de libération pré-chargé ;
▪ des moyens d'entraînement de forme appropriée en prises avec les blisters pour réaliser le déplacement de la bande de blisters après chaque inhalation, et amener un nouveau blister plein dans une position dans laquelle il est destiné à être ouvert par les moyens d'ouverture appropriés ;
▪ des moyens pour éviter une perte de dose en cas d'ouverture de l'inhalateur mais en l'absence d'inhalation ;
▪ un indicateur de doses adapté à compter les doses seulement en cas d'inhalation.

D'autres fonctions sont également fournies par l'inhalateur de l'invention tel que cela a été décrit précédemment.

Il est à noter que les différentes fonctions, même si elles ont été représentées comme prévues simultanément sur l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les blisters individuels sont arrangés les uns par rapport aux autres, etc. Les moyens d'armement et le système de déclenchement par l'inhalation pourraient être réalisés différemment. Il en est de même des autres parties constitutives du dispositif.

Diverses modifications sont également possibles pour un homme du métier sans sortir du cadre de la présente invention tel que défini par les revendications annexées. En particulier, les diverses caractéristiques et fonctionnalités du dispositif décrites en référence aux dessins peuvent être combinées entre elles d'une quelconque façon appropriée.

## Revendications

1. Inhalateur de poudre sèche comportant un corps principal (10), ledit inhalateur comportant une pluralité de réservoirs individuels (20), tels que des blisters, contenant chacun une dose unique de poudre, et disposés les uns derrière les autres sur une bande souple, des moyens d'ouverture (80) étant prévus pour ouvrir un réservoir individuel (20) à chaque actionnement de l'inhalateur, ledit inhalateur comportant des premiers moyens de déplacement (40) adaptés à amener un réservoir individuel (20) en face desdits moyens d'ouverture (80) avant et/ou après chaque actionnement, et des seconds moyens de déplacement (50) adaptés à déplacer un réservoir individuel (20) contre lesdits moyens d'ouverture (80) à chaque actionnement, lesdits premiers moyens de déplacement comportant une roue d'indexage (40) pourvue d'au moins un évidement (41) recevant un réservoir individuel, **caractérisé en ce que** ledit évidement (41) est défini par une paroi de fond (42) et une paroi latérale interne (43) de ladite roue d'indexage (40), et par une paroi latérale externe (420) formée sur un élément de réglage rapporté (400) assemblé sur ladite roue d'indexage (40).

2. Inhalateur selon la revendication 1, dans lequel ladite roue d'indexage (40) comporte une pluralité d'évidements (41).

3. Inhalateur selon la revendication 2, dans lequel ledit élément de réglage rapporté (400) comporte un manchon central (410) et une paroi externe annulaire (430), reliée audit manchon central (410) par une pluralité d'entretoises (420), lesdites entretoises (420) formant lesdites parois latérales externes desdits évidements (41) de la roue d'indexage.

4. Inhalateur selon l'une quelconque des revendications précédentes, dans lequel, les réservoirs individuels (20) sont des blisters, et dans lequel, après assemblage de l'élément de réglage rapporté (400) sur la roue d'indexage (40), la dimension des évidements (41) est sensiblement égale ou légèrement supérieure à la dimension extérieure des cavités (21) des blisters (20).

5. Inhalateur selon l'une quelconque des revendications précédentes, comportant un système de déclenchement par l'inhalation (60) qui comporte une chambre d'air déformable (61) coopérant avec un embout d'inhalation (200), et un élément déclencheur (600) coopérant avec ladite chambre d'air (61), de sorte que lors d'une inhalation à travers ledit embout d'inhalation (200), ladite chambre d'air (61) est déformée et ledit élément déclencheur (600) actionne lesdits moyens d'ouverture (80), de sorte que lors d'une inhalation à travers l'embout d'inhalation (200), un réservoir est ouvert par lesdits moyens d'ouverture (80).

6. Inhalateur selon l'une quelconque des revendications précédentes, dans lequel lesdits seconds moyens de déplacement (50) comportent des moyens de support mobiles (50) déplaçables entre une position de non-distribution et une position de distribution, lesdits moyens de support mobiles (50) étant sollicités vers leur position de distribution par des moyens élastiques (70), tel qu'un ressort ou une lame élastique, et étant retenus en position de non-distribution par des moyens de blocage qui sont libérés par l'inhalation de l'utilisateur.

7. Inhalateur selon la revendication 6, dans lequel ladite roue d'indexage (40) est montée rotative sur un axe (55) desdits moyens de support mobiles (50).

8. Inhalateur selon l'une quelconque des revendications précédentes, dans lequel lesdits moyens d'ouverture (80) comportent un élément de perçage fixe par rapport audit corps principal (10), adapté à découper une paroi de fermeture du réservoir de telle sorte que la ou les partie(s) découpée(s) n'obstrue(nt) pas la ou les ouverture(s) formée(s).

9. Inhalateur selon l'une quelconque des revendications précédentes, comportant au moins un élément de capot (11, 12) monté pivotant sur ledit corps principal (10) entre une position fermée et une position ouverte.

## Patentansprüche

1. Trockenpulverinhalator mit einem Hauptkörper (10), wobei der Inhalator eine Vielzahl von einzelnen Behältern (20), wie Blister, aufweist, die jeder eine Pulvereinzeldosis enthalten und hintereinander auf einem flexiblen Streifen angeordnet sind, wobei Öffnungseinrichtungen (80) vorgesehen sind, um einen einzelnen Behälter (20) bei jeder Betätigung des Inhalators zu öffnen, wobei der Inhalator erste Bewegungsmittel (40) aufweist, die eingerichtet sind, um einen einzelnen Behälter (20) vor und/oder nach jeder Betätigung vor die Öffnungseinrichtungen (80) zu bringen, und zweite Bewegungsmittel (50) aufweist, die eingerichtet sind, um einen einzelnen Behälter (20) bei jeder Betätigung gegen die Öffnungseinrichtungen (80) zu bewegen, wobei die ersten Bewegungsmittel ein Schaltrad (40) aufweisen, das mit mindestens einer Ausnehmung (41) zur Aufnahme eines Einzelbehälters versehen ist, **dadurch gekennzeichnet, dass** die Ausnehmung (41) durch eine Bodenwand (42) und eine innere Seitenwand (43) des Schaltrades (40) und durch eine äußere Seitenwand (420), die auf einem Einstelleinsatz (400) gebildet ist, der auf dem Schaltrad (40) montiert ist, definiert ist.

2. Inhalator nach Anspruch 1, wobei das Schaltrad (40) eine Vielzahl von Ausnehmungen (41) aufweist.

3. Inhalator nach Anspruch 2, wobei der Einstelleinsatz (400) eine mittlere Hülse (410) und eine ringförmige Außenwand (430) aufweist, die mit der mittleren Hülse (410) durch eine Vielzahl von Abstandshaltern (420) verbunden ist, wobei die Abstandshalter (420) die äußeren Seitenwände der Ausnehmungen (41) des Schaltrades bilden.

4. Inhalator nach einem der vorhergehenden Ansprüche, wobei die einzelnen Behälter (20) Blister sind und wobei das Maß der Ausnehmungen (41) nach der Montage des Einstelleinsatzes (400) auf dem Schaltrad (40) im Wesentlichen gleich oder geringfügig größer ist als das Außenmaß der Hohlräume (21) der Blister (20).

5. Inhalator nach einem der vorhergehenden Ansprüche, der ein System zum Auslösen durch Inhalation (60) aufweist, das eine verformbare Luftkammer (61) für das Zusammenwirken mit einem Inhalationsansatz (200) und ein Auslöseelement (600) für das Zusammenwirken mit der Luftkammer (61) aufweist, so dass bei einer Inhalation durch den Inhalationsansatz (200) die Luftkammer (61) verformt wird und das Auslöseelement (600) die Öffnungseinrichtungen (80) betätigt, so dass bei einer Inhalation durch den Inhalationsansatz (200) ein Behälter durch die Öffnungseinrichtungen (80) geöffnet wird.

6. Inhalator nach einem der vorhergehenden Ansprüche, wobei die zweiten Bewegungsmittel (50) bewegliche Trageeinrichtungen (50) aufweisen, die bewegbar sind zwischen einer Nichtausgabeposition und einer Ausgabeposition, wobei die beweglichen Trageeinrichtungen (50) durch elastische Einrichtungen (70), wie eine Feder oder eine Federlasche, in ihre Ausgabeposition beaufschlagt werden und durch Blockiereinrichtungen, die bei der Inhalation des Benutzers gelöst werden, in Nichtausgabeposition zurückgehalten werden.

7. Inhalator nach Anspruch 6, wobei das Schaltrad (40) auf einer Achse (55) der beweglichen Trageeinrichtungen (50) drehbar gelagert ist.

8. Inhalator nach einem der vorhergehenden Ansprüche, wobei die Öffnungseinrichtungen (80) ein Durchstoßelement aufweisen, das relativ zu dem Hauptkörper (10) festgelegt ist und eingerichtet ist, um eine Verschlusswand des Behälters so zu stanzen, dass der gestanzte Teil bzw. die gestanzten Teile die gebildete(n) Öffnung(en) nicht blockieren.

9. Inhalator nach einem der vorhergehenden Ansprüche, der mindestens ein Kappenelement (11, 12) aufweist, das auf dem Hauptkörper (10) zwischen einer geschlossenen Position und einer geöffneten Position drehbar gelagert ist.

## Claims

1. A dry powder inhaler including a main body (10), said inhaler including a plurality of individual reservoirs (20), such as blisters, each containing a single dose of powder, and disposed one behind another on a flexible strip, opening means (80) being provided for opening an individual reservoir (20) each time the inhaler is actuated, said inhaler including first displacement means (40) that are adapted to displace an individual reservoir (20) to face said opening means (80) before and/or after each actuation, and second displacement means (50) that are adapted to displace an individual reservoir (20) against said opening means (80) on each actuation, said first displacement means (40) comprising an indexer wheel provided with at least one recess (41) that receives an individual reservoir, said inhaler being **characterized in that** said recess (41) is defined by a bottom wall (42) and an inner side wall (43) of said indexer wheel (40), and by an outer side wall (420) formed on a separate adjustment element (400) assembled on said indexer wheel (40).

2. An inhaler according to claim 1, wherein said indexer wheel (40) includes a plurality of recesses (41).

3. An inhaler according to claim 2, wherein said separate adjustment element (400) comprises a central sleeve (410) and an annular outer wall (430) that is connected to said central sleeve (410) via a plurality of spacers (420), said spacers (420) forming said outer side walls of said recesses (41) of the indexer wheel.

4. An inhaler according to any preceding claim, wherein the individual reservoirs (20) are blisters, and wherein, after assembling the separate adjustment element (400) on the indexer wheel (40), the size of the recesses (41) is substantially equal to, or slightly greater than, the external size of the cavities (21) of the blisters (20).

5. An inhaler according to any preceding claim, including an inhalation trigger system (60) that comprises a deformable air chamber (61) that co-operates with an inhalation piece (200), and a trigger element (600) that co-operates with said air chamber (61), such that during inhalation through said inhalation piece (200), said air chamber (61) is deformed and said trigger element (600) actuates said opening means (80), such that during inhalation through the inhalation piece (200), a reservoir is opened by said opening means (80).

6. An inhaler according to any preceding claim, wherein said second displacement means (50) comprise movable support means (50) that are displaceable between a non-dispensing position and a dispensing position, said movable support means (50) being urged towards their dispensing position by resilient means (70), such as a spring or a spring blade, and being held in their non-dispensing position by blocking means that are released by the user inhaling.

7. An inhaler according to claim 6, wherein said indexer wheel (40) is rotatably mounted on a pin (55) of said movable support means (50).

8. An inhaler according to any preceding claim, wherein said opening means (80) include a perforator element that is stationary relative to said main body (10) and that is adapted to cut a closure wall of the reservoir in such a manner that the cut portion(s) does/do not obstruct the opening(s) that is/are formed.

9. An inhaler according to any preceding claim, including at least one cover element (11, 12) that is mounted to pivot on said main body (10) between a closed position and an open position.
